# EUROPÄISCHE PATENTSCHRIFT

(11) **EP 1 301 179 B1**
(45) Veröffentlichungstag und Bekanntmachung des Hinweises auf die Patenterteilung: **11.07.2007**
(21) Anmeldenummer: 01951670.7
(22) Anmeldetag: 12.07.2001
(51) Int. Cl.: A61K 9/70, A61K 47/02

(54) **TRANSDERMALES THERAPEUTISCHES SYSTEM MIT HOCHDISPERSEM SILIZIUMDIOXID**
TRANSDERMAL THERAPEUTIC SYSTEM FOR HIGHLY DISPERSED SILICON DIOXIDE
SYSTEME THERAPEUTIQUE TRANSDERMIQUE CONTENANT DU DIOXYDE DE SILICIUM FORTEMENT DISPERSE

(30) Priorität: 12.07.2000 DE 10033853
(43) Veröffentlichungstag der Anmeldung: 16.04.2003
(73) Patentinhaber: HEXAL AG, 83607 Holzkirchen (DE)
(72) Erfinder: KLOKKERS, Karin, 83620 Feldkirchen-Westerham (DE); KRAMER, Kai-Thomas, 83607 Holzkirchen (DE); WILHELM, Martina, 83607 Holzkirchen (DE)
(74) Vertreter: Held, Stephan
(86) Internationale Anmeldenummer: PCT/EP2001/008070
(87) Internationale Veröffentlichungsnummer: WO 2002/003969

(56) Entgegenhaltungen:
- EP-A- 0 259 136
- WO-A-93/08795
- DE-A- 19 827 732
- GB-A- 2 140 019

## Beschreibung

Die Erfindung betrifft hochdisperses Siliziumdioxid als penetrationsfördernde Substanz in wirkstoffhaltigen transdermalen therapeutischen Systemen, die einen ACE-Hemmer oder dessen pharmazeutisch unbedenkliche Salze als Wirkstoff enthalten.

Die transdermale Applikation bietet für eine Vielzahl von pharmazeutischen Wirkstoffen oder anderen biologisch aktiven Stoffen eine Reihe von Vorteilen:
die Haut ist unbegrenzt zugänglich,
es erfolgt kein Milieuwechsel wie bei der peroralen Applikation,
die Handhabung ist einfach und bequem,
es genügt normalerweise eine einmalige Gabe statt mehrfacher täglicher Gaben,
die Patienten-Compliance ist wesentlich besser,
es ist eine kontinuierliche Langzeittherapie möglich,
die Freisetzung des Wirkstoffes erfolgt annähernd gemäß einer Kinetik 0-ter Ordnung,
eine Therapie kann schneller unterbrochen werden,
es wird ein konstanter Plasmaspiegel über längere Zeit sichergestellt,
ein anfangs zu hoher Plasmaspiegel, wie bei intravenöser Applikation wird vermieden und es bedarf teilweise einer niedrigeren Dosierung als bei der oralen Gabe durch Umgehung der 1. Passage, wodurch eine geringere Nebenwirkungsrate auftritt,
die Gefahr von Über- oder Unterdosierung ist geringer,
es wird eine kontrollierte Abgabe von Wirkstoffen insbesondere mit niedrigem therapeutischen Index sichergestellt.

In vielen Fällen besitzen Wirkstoffe, die trotz ihrer niedrigen Dosierung und ihrer hohen Wirkpotenz ideale Kandidaten sind, eine derart geringe Hautpermeation, daß mit einfachen transdermalen therapeutischen Systemen eine Erzielung von therapeutischen Plasmaspiegeln nicht möglich ist. Für all diese Wirkstoffe ist es notwendig, dem System sogenannte Permeationsförderer zuzusetzen, um die Aufnahme in den systemischen Kreislauf zu erhöhen.

Penetrationsfördernde Substanzen müssen neben ihrer spezifischen Aufgabe folgende ideale Eigenschaften besitzen:

Sie müssen auch bei längerem Verbleib auf der Haut sowohl unter okklusiven als auch unter nicht-okklusiven Bedingungen hautverträglich sein, dürfen kein allergenisierendes Potential besitzen und müssen wirkstoffkompatibel sein.
Die aus der Literatur bekannten Enhancer lassen sich verschiedenen chemischen Klassen zuordnen:
1. Primäre und sekundäre Alkohole
   1.1 Kurzkettige primäre Alkohole C₂ bis C₈
   1.2 Langkettige primäre Alkohole C₄ bis C₁₆
   1.3 Sekundäre Alkohole C₃ bis C₂₀
2. Tenside
   2.1 Anionische Tenside wie z.B: Na-Dodecylsulfat
   2.2. Kationische Tenside (z.B. Cetylpyridiniumchlorid) bzw. Amine
3. Gesättigte und ungesättigte Fettsäuren
4. Azone und Derivate (1-Alkylazacycloheptan-2-on, 1-Alkylazacycloalkanon)
5. Amide wie N,N-Diethyl-3-methylbenzamid (DEET), N,N-Diethyl-m-toluamide
6. Alkyl-N,N-dialkylaminoacetat
7. Macrocyclische Ketone und Lactone
8. Pyrrolidone
9. Ester wie Etylacetat, Isopropylmyristat, Glycerinmonolaurat, Diethylsebacat, Propylenglykolester gesättigter Fettsäuren
10. Terpene wie Limonen, Menthol, Cineol
11. Phospholipide
12. Organische Säuren wie Citronensäure, Salicylsäure, etc.

Die Vielzahl unterschiedlicher Stoffe verschiedenster chemischer Struktur mit bekannter penetrationsfördemder Wirkung läßt einen einzigen Wirkungsmechanismus als unwahrscheinlich erscheinen. So werden dann auch verschiedene Mechanismen bzw. Kombinationen von Mechanismen diskutiert.
1. Lösemitteleffekte bezogen auf Wirkstoff und Hautlipide
2. Effekte auf die dreidimensionale Lipidstruktur der Membran
3. Effekte auf das Keratin und die Proteinstruktur der Haut

Bei der Vielzahl von Wechselwirkungen innerhalb der Haut und der unterschiedlichen chemischen Natur der Wirkstoffe lassen sich die penetrationsfördernden Eigenschaften all dieser Enhancer bezüglich eines Wirkstoffes nur schwer voraus sagen.

Erfahrungsgemäß gilt, daß äußerst selten eine penetrationsfördernde Substanz bzw. ein bestimmtes Gemisch für mehrere Wirkstoffe oder Wirkstoffgruppen die geforderten Eigenschaften erfüllt.

Aufgabe der Erfindung ist die Bereitstellung von einer penetrationsfördernden Substanz, die hautverträglich und wirkstoffkompatibel ist, sowie kein Allergenisierungspotential besitzt. Darüber hinaus sollte sie leicht zugänglich und wirtschaftlich sein und gleichzeitig eine penetrationsfördernde Wirkung auf mehr als auf einen Wirkstoff besitzen.

Überraschenderweise wurde nun gefunden, daß hochdisperses Siliziumdioxid die Eigenschaft aufweist, die Penetration von Wirkstoffen und / oder anderen biologisch aktiven Substanzen durch die Haut wesentlich zu erhöhen.

Hochdisperses Siliziumdioxid, auch bekannt unter dem Namen Aerosil® von der Firma Degussa-Hüls, wird üblicherweise zur Verdickung, Thixotropierung und Verstärkung verwendet. Aerosil bewirkt bei allen Elastomeren eine beträchtliche Verbesserung der mechanischen Eigenschaften, wie der Zug-, der Weiterreiß- oder Einreißfestigekeit. In feststoffhaltigen Flüssigkeiten, z.B. pigmentierten Lackfarben, verhindert oder verzögert Aerosil eine Sedimentation. Die Agglomeration und das Zusammenbacken der Feststoffteilchen von pulverförmigen Substanzen wird durch Aerosil als Fließhilfsmittel verhindert, so daß die Verpackung, Lagerung und Handhabung auch bei hoher Luftfeuchtigekeit und Druckbeanspruchung gewährleistet ist.
FR 5381 beschreibt Aerosil als Verdickungsmittel für topisch zu applizierende Salben.
DE 3416248 beschreibt den Zusatz von kolloidalen Siliziumdioxid in eine Pflastermatrix zur Viskositätserhöhung. FR 2547502 beschreibt eine Matrix für transdermale therapeutische Systeme, in der kolloidales Siliziumdioxid als thixotropes Agenz zugesetzt wird.
JP 2512565 B2 (Database Chemical Abstracts, AN 115:263477),
JP 2503095 B2 (Database Chemical Abstracts, AN 116:262553),
JP 06065066 (Database Chemical Abstracts, AN 121:42787) und
JP 04368323 (Database Chemical Abstracts, AN 118:175811) beschreiben Pflaster, bei denen Aerosil als Füllstoff eingesetzt wird.
JP 09169636 (Database Chemical Abstracts, AN 127:86139) beschreibt Siliziumdioxid in einem Pflaster zur Verringerung der Hautirritation.

Transdermale therapeutische Systeme sind ferner folgenden Dokumenten zu entnehmen: DE 44 05 898, DE 43 09 830, DE 42 30 588, WO 92/20 339; WO 91/05 529, US 5 939 090 und US 5 676 968. Dieser Stand der Technik gibt jedoch keine Anregung, mit Siliziumdioxid die Hautpermeation einzustellen.

DE 198 27 732 A und GB 2 140 019 A beschreiben Pflaster mit kolloidalem Siliziumdioxid, die eine erhöhte Wirkstofffreisetzung aufweisen.

Die Aufgabe wird erfindungsgemäß durch hochdisperses Siliziumdioxid gelöst, das als penetrationsfördernde Substanz in wirkstoffhaltigen oder in andere biologisch aktive Stoffe enthaltenden transdermalen therapeutischen Systemen wirkt.

Als hochdisperses Siliziumdioxid kann erfindungsgemäß Aerosil ^{®}90, Aerosil ^{®}130, Aerosil ^{®}150, Aerosil ^{®}200, Aerosil ^{®}300, Aerosil ^{®}380, Aerosil ^{®}OX50, Aerosil ^{®}TT600, Aerosil ^{®}MOX80, Aerosil ^{®}COK84, Aerosil ^{®}R202, Aerosil ^{®}R805, Aerosil ^{®}R812,
Aerosil ^{®}812S, Aerosil ^{®}R972 und/ oder Aerosil ^{®}R974 oder jedes andere hochdisperse Silizimdioxid, insbesondere Aerosil ^{®}200 und/ oder Aerosil ^{®}R972, verwendet werden.

Das transdermale therapeutische System, das den erfindungsgemäßen Permeationsförderer Siliziumdioxid enthält, stellt ein Pflaster dar. Bei diesem Pflaster kann es sich um ein Matrix- oder Membransystem handeln, welches eine undurchlässige Deckschicht und eine abziehbare Schutzschicht aufweist. Bei einem Membransystem liegt das Reservoir bzw. die Reservoirschicht zwischen der Deckschicht und der Membran.

Als undurchlässige Deckschicht kommen Folien aus Acetal, Acrylat, Acrylonitril-Butadien-Styrol, Acrylonitril (Methyl Methacrylat) Copolymer, Acrylonitril Copolymer, Ethylen Ethyl Acrylat, Ethylen Methyl Acrylat, Ethylen Vinyl Acetat, Ethylen Vinyl Acetat Copolymer, Ethylen Vinylalkohol Polymer, Ionomere, Nylon (Polyamid), Nylon (Polyamid) Copolymer, Polybutylen, Polycarbonat, Polyester, Polyethylenterephthalat, thermoplastisches Polyester Copolymer, Polyethylen Copolymer (high density), Polyethylen (high-molecular-weight, high-density), Polyethylen (intermediate-molecular-weight, high-density), Polyethylen(linear low density), Polyethylen (low density), Polyethylen (medium density), Polyethylenoxid, Polyimid, Polypropylen, Polypropylen (coated), Polypropylen (oriented), Polystyrol, Polyurethan, Polyvinylacetat, Polyvinylchlorid, Polyvinylidenchlorid und/ oder Styrol-Acrylonitril in Frage, die bei Bedarf metallisiert oder pigmentiert werden können.

Für die abziehbare Schutzschicht kommen Polyester, Polyethylen, Polypropylen, Polysiloxan, Polyacrylat, Ethylenvinylacetat, Polyurethan, Polyisobuten oder Papier, meistens mit Silikon- und /oder Polyethylen beschichtet, oder ein Gemisch aus diesen in Betracht.

Ein Matrixpflaster besteht aus einer für den Wirkstoff undurchlässigen Deckschicht, aus einer oder mehreren den Wirkstoff enthaltenden, selbstklebenden Matrixschicht(en) oder einer oder mehreren Matrixschicht(en), die mit einem Haftkleber beschichtet sind und einer abziehbaren Schutzschicht.
Für die Matrix werden die medizinisch üblichen Matrixbildner wie Polyacrylat, Silikon, Polyisobutylen, Kautschuk, kautschukähnliche synthetische Homo-, Co- oder Blockpolymere, Butylkautschuk, Styrol/ Isopren- Copolymerisat, Polyurethane, Copolymere des Ethylens, Polysiloxane, Styrol/ Butadien- Copolymerisat oder ein Gemisch aus diesen, wie sie im Stand der Technik vorgesehen werden, verwendet.
Als Kleber kann Polydimethylsiloxan, Polyacrylate, Polyisobutylen, Polyacrylat mit C₄-C₁₀ Alkylalkoholestern, amminrestistentes Silikon in Ethylacetat oder n-Heptan, Polyisobutylen/ Mineralöl oder ein Gemisch aus diesen verwendet werden.

Eine weitere erfindungsgemäße Ausführungsform stellt ein Membransystem dar. Dieses besteht aus einer für den Wirkstoff undurchlässigen Deckschicht, einem wirkstoffhaltigen Reservoir oder einer Reservoirschicht, einer semipermeablen Membran, einer Haftklebeschicht und einer abziehbaren Schutzschicht.

Die Matrix (die Matrizes) oder das Reservoir enthält den oder die Wirkstoff(e) und gegebenenfalls Stabilisatoren, Emulgatoren, Verdickungsmittel, Permeationsförderer und/ oder übliche Matriz-, Membransystem- bzw. Reservoirpflaster- Hilfsmittel. Als Gelbildner können bei Bedarf Cellulosederivate, wie z.B. Methylcellulose, Hydroxypropylcellulose, Hydroxyethylcellulose oder Carboxymethylcellulose, und/ oder Carboxyvinylpolymer, Polyacrylate, Natrium- Plyoxilat oder ein Gemisch aus diesen verwendet werden.
Die Membran, die üblicherweise aus inerten Polymeren, insbesondere auf Basis von Polyethylen, Polypropylen, Polyvinylacetat, Polyamid, Ethylen- Vinylacetat- Copolymeren und/ oder Silikon, besteht, kann je nach Porengröße eine die Wirkstoffreisetzung kontrollierende Wirkung haben.

Für die Haftklebeschicht kann man ein druckempfindliches Klebemittel beispielsweise auf Polyurethanbasis, Polyisobutylenbasis, Polyvinyletherbasis, Siliconbasis, Polyacrylatbasis oder ein Gemisch aus diesen wählen.

Bei dem Klebemittel auf Silkonbasis kann es sich um Silikonkleber handeln, welche auf zwei Hauptbestandteilen basieren: Ein Polymer oder Klebstoff, insbesondere Polysiloxan, und ein die Klebrigkeit erhöhendes Harz. Der Polysiloxankleber ist gewöhnlich mit einem Vernetzer für den Kleber, typischerweise mit einem hochmolekularen Polydiorganosiloxan, und mit dem Harz zubereitet, um über ein angemessenes organisches Lösungsmittel eine dreidimensionale Silikatstruktur zu ergeben. Die Zumischung des Harzes zu Polymer ist der wichtigste Faktor, um die physikalischen Eigenschaften der polysiloxanen Kleber zu ändern; vgl. beispielsweise Sobieski, et al., "Silicone Pressure Sensitive Adhesives", Handbook of Pressure Sensitive Adhesive Technology, 2nd ed., pp. 508-517 (D. Satas, ed.), Van Nostrand Reinhold, New sive Technology, 2nd ed., pp. 508-517 (D. Satas, ed.), Van Nostrand Reinhold, New York (1989).

Ein weiteres Beispiel für ein druckempfindliches Klebemittel auf Silikonbasis ist trimethyliertes Siliciumdioxid, das mit Polydimethylsiloxan mit endständigen Trimethylsiloxy- Gruppen behandelt worden ist.

Bei den Klebemitteln auf Polyacrylatbasis kann es sich um ein beliebiges Homopolymer, Copolymer oder Terpolymer, bestehend aus verschiedenen Acrylsäurederivaten handeln.

So können die Polyacrylate Polymere eines oder mehrerer Monomere von Acrylsäuren und anderen copolymerisierbaren Monomeren sein. Außerdem können die Acrylatpolymere Copolymere von Alkylacrylaten und/ oder -methacrylaten und/ oder copolymerisierbaren sekundären Monomeren oder Monomeren mit funktionellen Gruppen umfassen. Verändert man den Betrag jeder Sorte, die als Monomer hinzugefügt ist, können die kohäsiven Eigenschaften der daraus resultierenden Acrylatpolymere verändert werden. Im allgemeinen besteht das Acrylatpolymer aus mindestens 50 Gew.-% eines Acrylat-, Methacrylat-, Alkylacrylat- oder Alkylmethacrylat-Monomers, 0 bis 20 % eines funktionellen Monomers, copolymerisierbar mit Acrylat, und 0 bis 50 % eines anderen Monomeren.

Im folgenden sind verschiedene Acrylatmonomere, wie z.B. Acrylsäure, Methacrylsäure, Butylacrylat, Butylmethacrylat, Hexylacrylat, Hexylmethacrylat, Isooctylacrylat, Isooctylmethacrylat, Glycidylmethacrylat, 2-Hydroxyethylacrylat, Methylacrylat, Methylmethacrylat, 2-Ethylhexylacrylat, 2-Ethylhexylmethacrylat, Decylacrylat, Decylmethacrylat, Dodecylacrylat, Dodecylmethacrylat, Tridecylacrylat und Tridecylmethacrylat, aufgeführt, die alleine oder in Mischung polymerisiert werden können.

Zusätzlich können funktionelle Monomere, die mit den oben genannten Acrylaten copolymerisierbar sind, wie beispielsweise Acrylsäure, Methacrylsäure, Maleinsäure, Maleinanhydrid, Hydroxyethylacrylat, Hydroxypropylacrylat, Acrylamid, Dimethylacrylamid, Acrylnitril, Dimethylaminoethylacrylat, Dimethylaminoethylmethacrylat, tert.-Butylaminoethylacrylat, tert.-Butylaminoethylmethacrylat, Methoxyethylacrylat, Vinylacetat und Methoxyethylmethacrylat, zur Copolymerisierung eingesetzt werden.

Weiter Einzelheiten und Beispiele für druckempfindliche Acrylate, welche für die Erfindung geeignet sind, sind in Satas Handbook of Pressure Sensitive Adhesive Technology "Acrylic Adhesives", 2nd ed., pp. 396-456 (D. Satas, ed.), Van Nostrand Reinhold, New York (1959) beschrieben.

Das erfindungsgemäße penetrationsfördernde hochdisperse Siliziumdioxid liegt in der Kleberschicht eingearbeitet vor. Es ist in dieser homogen verteilt. Um eine homogene Verteilung zu erlangen, muß das Siliziumdioxid im allgemeinen mit dem Klebstoff bei der Einarbeitung quellen. Während der Quellphase wird das Kleber-Siliziumdioxid-Gemisch mit einem geeigneten Gerät z.B. mit einem Ultraturax über eine längere Zeit durchmischt.

Der Gehalt an hochdispersem Siliziumdioxid bezogen auf das Matrixgewicht der Klebeschicht des transdermalen therapeutischen Systems kann 0,1-10 Gew.%, insbesondere 2-5 Gew.% und bevorzugt 2,4 oder 5 Gew.% betragen.

Der in dem transdermalen therapeutischen System enthaltene Wirkstoff ist ein Vertreter aus der Wirkstoffgruppe der ACE-Hemmer

Das transdermale therapeutische System enthält einen oder mehrere Vertreter aus der Gruppe der ACE-Hemmer z.B. Alacepril, Benazepril, Ceronapril, Cilazapril, Denapril, Fosinopril, Imidapril, Moexipril, Moveltipril, Perindopril, Quinapril, Ramipril, Ramiprilat, Rentiapril, Spirapril, Temocapril, Trandolapril, Utibapril, Zofenopril und/oder deren Ester und/ oder deren weitere pharmazeutisch unbedenklichen Salze als Wirkstoffkomponente.

Unter pharmazeutisch unbedenklichen Salzen der genannten basischen Wirkstoffe werden Säureadditionssalze verstanden. Diese erhält man durch die Reaktion des in der freien Basenform vorliegenden Wirkstoffes mit pharmazeutisch unbedenklichen Säuren. Pharmazeutisch unbedenkliche Säuren sind anorganische Säuren (z.B. Salzsäure, Bromwasserstoffsäure, Schwefelsäure, Salpetersäure, Phosphorsäure) oder organische Säuren (z.B. Essig-, Propion-, Hydroxyessig-, Milch-, Brenztrauben-, Oxal-, Malein-, Malon-, Bernstein-, Fumar-, Äpfel-, Wein-, Citronen-, Methansulfon-, Ethansulfon-, Benzolsulfon-, p-Toluolsulfon-, Cyclohexansulfamin-, Salicyl-, p-Aminosalicyl- und Pamoasäure). Ebenso als Säureadditionssalze werden Solvate mit dem Wirkstoff bezeichnet. Derartige Solvate sind z.B. Hydrate, Alkoholate und dergleichen. Als mögliche pharmazeutisch unbedenkliche Salze der genannten sauren Wirkstoffe kommen vor allem Alkalimetall- und/ oder Erdalkalimetallsalze sowie das Ammoniumsalz in Frage wie z.B. das Kalium-, Natrium-, Lithium-, Calcium-, Magnesium- und Ammoniumsalz.

Bevorzugt werden gut wasserlösliche Wirkstoffe, und/ oder deren pharmazeutisch unbedenklichen Salze in dem transdermalen therapeutischen System als Wirkstoffkomponente verwendet.

In dem Reservoir bzw. in der Matrixschicht können neben dem Wirkstoff gegebenenfalls weitere nach dem Stand der Technik bekannte Penetrationsförderer enthalten sein, wenn die Penetration des Wirkstoffes durch die Haut bei Abwesenheit des erfindungsgemäßen Siliziumdioxids im transdermalen therapeutischen System nicht ausreichend hoch ist. Als zusätzliche Permeationsförderer lassen sich ein- und/oder mehrwertige aliphatische, cycloaliphatische und /oder aromatisch- aliphatische Alkohole mit jeweils bis zu acht C- Atomen, z.B. Ethanol, 1,2-Propandiol, Dexpanthenol und/ oder Polyethylenglykol; Alkohol/ Wasser- Gemische; gesättigte und/ oder ungesättigte Fettalkohole mit jeweils 8- 18 C- Atomen; Terpene; z.B. Cineol, Carveol, Menthon, Terpineol, Verbenon, Menthol, Limonen, Thymol, Cymen, Terpinen-4-ol, Neomenthol, Geraniol, Fenchon; Gemische aus Terpenen und Etanol und/ oder Propylenglykol; Teebaumöl; gesättigte und/ oder ungesättigte cyclische Ketone; Alkyl- Methylsulfoxide; gesättigte und/ oder ungesättigte Fettsäuren mit jeweils 8- 18 C- Atomen; deren Ester und Salze; natürliches Vitamin E; synthetisches Vitamin E und/ oder Vitamin E- Derivate; Sorbitanfettsäureester und ethoxylierte Sorbitanfettsäureester; Azone (Laurocapram); Azone gemischt mit Alkoholen; Harnstoff; 1-Alkylpyrrolidon; Blockcopolymere von Polyethylenglykol und Dimethylsiloxan mit kationischer Gruppe an einem Ende; Isopropylmyristat, Isopropylpalmitat, Folat-Polyethylenglykol-Liposom, Proliposom; Polyoxyethylen-10-stearylether; Gemisch aus Polyoxyethylen-10-stearylether und Glyceryldilaurat; Dodecyl-2-(N,N-dimethylamino)-propanoltetradecanoat und/ oder Dodecyl-2-(N,N-dimethylamino)-propianat; N-Acetylprolinatester mit > 8 C-Atomen; nichtionische Tenside, z.B. Laurylether, Ester von Polyoxyethylen; Ethosom (Phospholipidvesikel); Dimethyl(arylimino)sulfuran; Gemisch aus Ölsäureanaloga und Propylenglykol; Gemisch aus Padimat O, Oktylsalicylat, Oktylmethoxycinnimat, Laurocapram; Cetiol^{®} HE; Eutanol® G oder ein Gemisch aus Einzelkomponenten verwenden.

Die Erfindung wird durch nachstehende Beispiele näher erläutert.

### Beispiel 1:

Vergleich der Permeationswerte eines transdermalen therapeutischen Systems (TTS) **mit** und **ohne** Siliziumdioxid. Es wurde ein Polyisobutylenkleber (MA24A^{®} von AdhesiveResearch' USA) und Trandolapril als Wirkstoff verwendet.

Apparatur für die Hautpermeation:

| | |
|---|---|
| Zellen: | modifizierte Durchflußzelle |
| Haut: | Hairless mouse von weiblichen Mäusen, |
| Akzeptormedium: | 0,9 % Natriumchlorid + 0,05 % Natriumazid, 60 ml pro Zelle |
| Permeationstemp.: | 32 °C ± 0,5 °C |

Die Wirkstoffkonzentrationen werden dann nach dem Probenzug mittels HPLC bestimmt.

| TTS ohne Siliziumdioxid in der Matrix | Permeationswerte [µg/cm²] | |
|---|---|---|
| Trandolapril 10 Gew. % | 24 h | 4,9-14,4 |
| Eutanol^{®} G 10 Gew. % | 48 h | 11,9-26,6 |
| MA24A^{®} 80 Gew. % | | |

| TTS mit Siliziumdioxid in der Matrix | Permeationswerte [µg/cm²] | |
|---|---|---|
| Trandolapril 10 Gew. % | 24 h | 37,6-58,0 |
| Eutanol^{®} G 10 Gew. % | 48 h | 82,6-118,1 |
| MA24A^{®} 76 Gew. % | | |
| Aerosil^{®} 200 4 Gew. % | | |

| | | |
|---|---|---|
| Die Gewichtsprozente sind auf das Matrixgewicht bezogen. | | |

### Beispiel 2:

Vergleich der Permeationswerte eines transdermalen therapeutischen Systems (TTS) mit und ohne Siliziumdioxid. Es wurde ein Polyisobutylenkleber (MA24A^{®}) und das Methansulfonsäure-Salz von Trandolapril als Wirkstoff verwendet, wobei Trandolaprilmesylat in-situ im TTS gebildet wird.

Apparatur für die Hautpermeation:

| | |
|---|---|
| Zellen: | modifizierte Durchflußzelle |
| Haut: | Hairless mouse von weiblichen Mäusen |
| Akzeptormedium: | 0,9 % Natriumchlorid + 0,05 % Natriumazid, 60 ml pro Zelle |
| Permeationstemp.: | 32 °C ± 0,5 °C |

| TTS ohne Siliziumdioxid in der Matrix | Permeationswerte [µg/cm²] | |
|---|---|---|
| Trandolapril 10 Gew. % | 24 h | 10,9 - 13,8 |
| Methansulfonsäure 2,26 Gew. % | 48 h | 19,2-24,6 |
| Eutanol^{®} G 5 Gew. % | | |
| MA24A^{®} 78,7 Gew. % | | |

| TTS mit Siliziumdioxid in der Matrix | Permeationswerte [µg/cm²] | |
|---|---|---|
| Trandolapril 10 Gew. % | 24 h | 36,1 - 74,4 |
| Methansulfonsäure 2,26 Gew. % | 48 h | 146,2 - 204,6 |
| Eutanol^{®} G 5 Gew. % | | |
| MA24A^{®} 78,74 Gew. % | | |
| Aerosil^{®} 200 4 Gew. % | | |

| | | |
|---|---|---|
| Die Gewichtsprozente sind auf das Matrixgewicht bezogen. | | |

### Beispiel 3:

Vergleich der Permeationswerte eines transdermalen therapeutischen Systems (TTS) mit und ohne Siliziumdioxid. Es wurde ein Polyisobutylenkleber (MA24A^{®}) und Ramiprilmesylat als Wirkstoff verwendet.

Apparatur für die Hautpermeation:

| | |
|---|---|
| Zellen: | modifizierte Durchflußzelle |
| Haut: | Hairless mouse von weiblichen Mäusen |
| Akzeptormedium: | 0,9 % Natriumchlorid + 0,05 % Natriumazid, 60 ml pro Zelle |
| Permeationstemp.: | 32 °C ± 0,5 °C |

| TTS ohne Siliziumdioxid in der Matrix | Permeationswerte [µg/cm²] | |
|---|---|---|
| Ramiprilmesylat 15 Gew. % | 24 h | 111 - 156 |
| Eutanol^{®} G 10 Gew. % | 48 h | 184 - 219 |
| MA24A^{®} 75 Gew. % | | |

| TTS mit Siliziumdioxid in der Matrix | Permeationswerte [µg/cm²] | |
|---|---|---|
| Ramiprilmesylat 15 Gew. % | 24 h | 251-338 |
| Eutanol^{®} G 10 Gew. % | 48 h | 862-997 |
| MA24A^{®} 70 Gew. % | | |
| Aerosil^{®} 200 5 Gew. % | | |

| | | |
|---|---|---|
| Die Gewichtsprozente sind auf das Matrixgewicht bezogen. | | |

### Beispiel 4:

Vergleich der Permeationswerte eines transdermalen therapeutischen Systems (TTS) mit und ohne Siliziumdioxid. Es wurde ein Acrylatkleber (Durotak^{®} von National Starch/ USA) und Ramiprilmesylat als Wirkstoff verwendet, wobei Ramiprilmesylat in-situ gebildet wird

Apparatur für die Hautpermeation:

| | |
|---|---|
| Zellen: | modifizierte Durchflußzelle |
| Haut: | Hairless mouse von weiblichen Mäusen |
| Akzeptormedium: | 0,9 % Natriumchlorid + 0,05 % Natriumazid, 60 ml pro Zelle |
| Permeationstemp.: | 32 °C ± 0,5 °C |

| TTS ohne Siliziumdioxid in der Matrix | Permeationswerte [µg/cm²] | |
|---|---|---|
| Ramipril 15 Gew. % | 24 h | 6-29 |
| Methansulfonsäure 3,8 Gew. % | 48 h | 11-53 |
| Eutanol^{®} G 10 Gew. % | | |
| Durotak^{®} 387-2353 71,2 Gew. % | | |

| TTS mit Siliziumdioxid in der Matrix | Permeationswerte [µg/cm²] | |
|---|---|---|
| Ramipril 15 Gew. % | 24 h | 122-248 |
| Methansulfonsäure 3,5 Gew. % | 48 h | 366-533 |
| Eutanol^{®} G 10 Gew. % | | |
| Durotak^{®} 387-2353 66,5 Gew. % | | |
| Aerosil^{®} 200 5 Gew. % | | |

| | | |
|---|---|---|
| Die Gewichtsprozente sind auf das Matrixgewicht bezogen. | | |

### Beispiel 5:

Vergleich der Permeationswerte eines transdermalen therapeutischen Systems (TTS) mit und ohne Siliziumdioxid. Es wurde ein Acrylatkleber (Durotak^{®}) und Ramiprilmesylat als Wirkstoff verwendet, wobei Ramiprilmesylat in-situ gebildet wird.

Apparatur für die Hautpermeation:

| | |
|---|---|
| Zellen: | modifizierte Durchflußzelle |
| Haut: | Hairless mouse von weiblichen Mäusen, |
| Akzeptormedium: | 0,9 % Natriumchlorid + 0,05 % Natriumazid, 60 ml pro Zelle |
| Permeationstemp.: | 32 °C ± 0,5 °C |

| TTS ohne Siliziumdioxid in der Matrix | Permeationswerte [µg/cm²] | |
|---|---|---|
| Ramipril 10 Gew. % | 24 h | 19-24 |
| Methansulfonsäure 2,4 Gew. % | 48 h | 41-48 |
| Eutanol^{®} G 10 Gew. % | | |
| Durotak^{®} 387-2510 77,6 Gew. % | | |

| TTS **mit** Siliziumdioxid in der Matrix | Permeationswerte [µg/cm²] | |
|---|---|---|
| Ramipril 10 Gew. % | 24 h | 51-80 |
| Methansulfonsäure 2,4 Gew. % | 48 h | 205-253 |
| Eutanol^{®} G 10 Gew. % | | |
| Durotak^{®} 387-2510 73,6 Gew. % | | |
| Aerosil^{®} 200 4 Gew. % | | |

| | | |
|---|---|---|
| Die Gewichtsprozent sind auf das Matrixgewicht bezogen. | | |

### Beispiel 6: (nicht gemäß der Erfindung)

Vergleich der Permeationswerte eines transdermalen therapeutischen Systems (TTS) mit und ohne Siliziumdioxid. Es wurde ein Polyisobutylenkleber (MA24A^{®}, Moxonidin-Base als Wirkstoff und Ölsäure als Enhancer verwendet.

Apparatur für die Hautpermeation:

| | |
|---|---|
| Zellen: | modifizierte Durchflußzelle |
| Haut: | Hairless mouse von weiblichen Mäusen, |
| Akzeptormedium: | 0,9 % Natriumchlorid + 0,05 % Natriumazid, 60 ml pro Zelle |
| Permeationstemp.: | 32 °C ± 0,5 °C |

| TTS ohne Siliziumdioxid in der Matrix | Permeationswerte [µg/cm²] | |
|---|---|---|
| Moxonidin 10 Gew. % | 24 h | 23,0-42,6 |
| Ölsäure 10 Gew. % | 48 h | 45,3-82,1 |
| MA24A^{®} 80 Gew. % | | |

| TTS mit Siliziumdioxid in der Matrix | Permeationswerte [µg/cm²] | |
|---|---|---|
| Moxonidin 10 Gew. % | 24 h | 58,6-84,8 |
| Ölsäure 10 Gew. % | 48 h | 107,8-159,0 |
| Aerosil^{®} 200 3 Gew. % | | |
| MA24A^{®} 77 Gew. % | | |

| | | |
|---|---|---|
| Die Gewichtsprozent sind auf das Matrixgewicht bezogen. | | |

### Beispiele 7 und 8:

Für Ramipril Mesilat sollte eine Hautpermeation mit Eutanol G von etwa 920 µg / cm² / 48h (Beispiel 7) und mit Eutanol G /α-Tocopherolacetat von etwa 680µg / cm²/ 48h (Beispiel 8) eingestellt werden. Diese Vorgaben wurden durch Erhöhung des Gehaltes an Aerosil 200 erreicht; vgl, die folgende Tabelle.

| Charge (Ramipril-TTS) | Zusammensetzung | Hautperm [µg/cm²] |
|---|---|---|
| RAM 0076 TTS | Ramipril Mesilat 15 % | 24h: 294, 202 |
| | Eutanol G 10 % | |
| | Aerosil 200 2 % | 48h: 509, 394 |
| | MA 24 73 % | |
| RAM 0081 TTS | Ramipril Mesilat 15 % | 24h: 251, 338 |
| | Eutanol G 10 % | |
| | Aerosil 200 5 % | 48h: 997, 862 |
| | MA 24 70 % | |
| RAM 0077 TTS | Ramipril Mesilat 15 % | 24h: 148, 99 |
| | Eutanol G 10% | |
| | α-Tocopherolacetat. 10% | 48h: 313,272 |
| | Aerosil 200 2 % | |
| | MA 24 63 % | |
| RAM 0083 TTS | Ramipril Mesilat 15% | 24h: 187, 190 |
| | Eutanol G 10 % | |
| | α-Tocopherolacetat. 10% | 48h: 653, 708 |
| | Aerosil 200 5 % | |
| | MA 24 60 % | |

## Patentansprüche

1. Transdermales therapeutisches System mit einer wirkstoffundurchlässigen Deckschicht, mit einer selbstklebenden Matrixschicht oder mit mehreren Matrixschichten, von denen mindestens die bei Applikation des Systems freiliegende Matrixschicht selbstklebend ist, und.mit einer abziehbaren Schutzschicht, wobei die Matrixschicht(en) einen oder mehrere Wirkstoffe und/oder einen oder mehrere andere biologisch aktive Stoffe und hochdisperses Siliziumdioxid enthält (enthalten), wobei das System Siliziumdioxid zur Erhöhung der Hauptpermeation enthält, und der (die) in dem transdermalen therapeutischen System enthaltene(n) Wirkstoff(e) ein ACE-Hemmer oder dessen pharmazeutisch unbedenkliche Salze ist (sind).

2. Transdermales therapeutisches System mit einer wirkstoffundurchlässigen Deckschicht, mit einer oder mehreren Matrixschichten mit einem Gehalt an Wirkstoff und/oder anderen biologisch aktiven Stoffen, wobei die Matrixschicht(en) mit einem Haftkleber (Klebeschicht) beschichtet ist (sind), und mit einer abziehbaren Schutzschicht wobei die Klebeschicht hochdisperses Siliziumdioxid enthält und der in dem transdermalen therapeutischen System enthaltene Wirkstoff ein ACE-Hemmer oder dessen pharmazeutisch unbedenkliche Salze ist (sind).

3. Transdermales therapeutisches System nach Anspruch 2, **dadurch gekennzeichnet, daß** das System Siliziumdioxid zur Erhöhung der Hautpermeation enthält.

4. Transdermales therapeutisches System mit einer wirkstoffundurchlässigen Deckschicht, einem Reservoir oder einer Reservoirschicht mit einem Gehalt an Wirkstoff und/oder anderen biologisch aktiven Stoffen, mit einer semipermeablen Membran, mit einer Klebeschicht und mit einer abziehbaren Schutzschicht, wobei die Klebeschicht hochdisperses Siliziumdioxid zur Erhöhung der Hautpermeation enthält und der (die) in dem transdermalen therapeutischen System enthaltene(n) Wirkstoff(e) ein ACE-Hemmer, dessen pharmazeutisch unbedenkliche Salze ist (sind).

5. Transdermales therapeutisches System nach einem der vorhergehenden Ansprüche, **dadurch gekennzeichnet, daß** das System. Siliziumdioxid in einer Menge enthält, die bei einem (abgesehen von Siliziumdioxid) vorgegebenen System einem vorgegebenen Permeationswert entspricht oder auf einen vorgegebenen Permeationswert eingestellt ist.

6. Transdermales therapeutisches System nach Anspruch 5, **gekennzeichnet durch** einen Siliziumdioxid-Gehalt, der einem maximalen Permeationswert entspricht.

7. Transdermales therapeutisches System nach einem der vorhergehenden Ansprüche, **dadurch gekennzeichnet, daß** das hochdisperse Silizumdioxid in die selbstklebende Matrixschicht(en) oder in die Klebeschicht homogen eingearbeitet worden ist:

8. Transdermales therapeutisches System nach Anspruch 7, **dadurch gekennzeichnet, daß** das hochdisperse Siliziumdioxid eingearbeitet worden ist, indem man ein Gemisch aus selbstklebender Matrix/Siliziumdioxid oder ein Gemisch aus Haftkleber/Siliziumdioxid in Gegenwart eines flüssigen Mediums quellen ließ und danach zu Matrixschicht(en) oder Klebeschicht ausformte.

9. Transdermales therapeutisches System nach einem der vorhergehenden Ansprüche, **gekennzeichnet durch** einen Gehalt an hochdispersem Siliziumdioxid von 0,1 bis 10 Gew.-%, insbesondere 2 bis 5 Gew.-% und bevorzugt etwa 2, etwa 3, etwa 4 oder etwa 5 Gew.%, bezogen auf das Gewicht der selbstklebenden Matrixschicht(en) oder der Klebeschicht.

10. Transdermales therapeutisches System nach einem der vorhergehenden Ansprüche, **gekennzeichnet durch** Aerosil*90, Aerosil* 130, Aerosil* 150, Aerosil* 200, Aerosil*300, Aerosil* 380, Aerosil* 0x50, Aerosil* TT600, Aerosil* MOX8O, Aerosil* COK84, Aerosil* R202, Aerosil* R805, Aerosil* R812, Aerosil* 812S, Aerosil* R972 und/oder Aerosil* R974 als hochdisperses Siliziumdioxid.

11. Transdermales therapeutisches System nach Anspruch 10, **gekennzeichnet durch** Aerosil* 200 und/oder Aerosil* R972 als hochdisperses Siliziumdioxid.

12. Transdermales therapeutisches System nach einem der vorhergehenden Ansprüche, **gekennzeichnet durch** einen Gehalt an Stabilisatoren, Emulgatoren, Verdickungsmitteln, Permeationsförderern und/oder üblichen Membransystem- oder Reservoirpflaster-Hilfsmitteln.

13. Verwendung von hochdispersem Siliziumdioxid als Penetrationsförderer in einem transdermalen therapeutischen System mit einer wirkstoffundurchlässigen Deckschicht, mit einer selbstklebenden Matrixschicht oder mit mehreren Matrixschichten, von denen mindestens die bei Applikation des Systems freiliegende Matrixschicht selbstklebend ist, und mit einer abziehbaren Schutzschicht, wobei die Matrixschicht(en) einen oder mehrere Wirkstoffe und/oder einen oder mehrere andere biologisch aktive Stoffe und hochdisperses Siliziumdioxid enthält (enthalten); wobei der (die) in dem transdermalen therapeutischen System enthaltene(n) Wirkstoff(e) ein ACE-Hemmer, oder dessen pharmazeutisch unbedenkliche Salze ist (sind).

14. Verwendung von hochdispersem Siliziumdioxid als Penetrationsförderer in einem transdermalen therapeutischen System mit einer wirkstoffundurchlässigen Deckschicht, mit einer oder mehreren Matrixschichten mit einem Gehalt an Wirkstoff und/oder anderen biologisch aktiven Stoffen, wobei die Matrixschicht(en) mit einem Haftkleber (Klebeschicht) beschichtet ist (sind), und mit einer abziehbaren Schutzschicht, wobei die Klebeschicht hochdisperses Siliziumdioxid enthält und der (die) in dem transdermalen therapeutischen System enthaltene(n) Wirkstoff(e) ein ACE-Hemmer, oder dessen pharmazeutisch unbedenkliche Salze ist (sind).

15. Verwendung von hochdispersem Siliziumdioxid als Penetrationsförderer in einem transdermalen therapeutischen System mit einer wirkstoffundurchlässigen Deckschicht, einem Reservoir oder einer Reservoirschicht mit einem Gehalt an Wirkstoff und/oder anderen biologisch aktiven Stoffen, mit einer semipermeablen Membran, mit einer Klebeschicht und mit einer abziehbaren Schutzschicht, wobei die Klebeschicht hochdisperses Siliziumdioxid zur Erhöhung der Hautpermeation enthält und der (die) in dem transdermalen therapeutischen System enthaltene(n) Wirkstoff(e) ein ACE-Hemmer order dessen pharmazeutisch unbedenkliche Salze ist (sind).

16. Verwendung nach einem der vorhergehenden Ansprüche 13 bis 15, **dadurch gekennzeichnet, daß** das System Siliziumdioxid in einer Menge enthält, die bei einem (abgesehen von Siliziumdioxid) vorgegebenen System einem vorgegebenen Permeationswert entspricht oder auf einen vorgegebenen Permeationswert eingestellt ist.

17. Verwendung nach Anspruch 16, **gekennzeichnet durch** einen Siliziumdioxid-Gehalt, der einem maximalen Permeationswert entspricht.

18. Verwendung nach einem der vorhergehenden Ansprüche 13 bis 17, **dadurch gekennzeichnet, daß** das hochdisperse Silizumdioxid in die selbstklebende Matrixschicht(en) oder in die Klebeschicht homogen eingearbeitet worden ist.

19. Verwendung nach Anspruch 18, **dadurch gekennzeichnet, daß** das hochdisperse Siliziumdioxid eingearbeitet worden ist, indem man ein Gemisch aus selbstklebender Matrix/Siliziumdioxid oder ein Gemisch aus Haftkleber/Siliziumdioxid in Gegenwart eines flüssigen Mediums quellen ließ und danach zu Matrixschicht(en) oder Klebeschicht ausformte.

20. Verwendung nach einem der vorhergehenden Ansprüche 13 bis 19, **gekennzeichnet durch** einen Gehalt an hochdispersem Siliziumdioxid von 0,1 bis 10 Gew.-%, insbesondere 2 bis 5 Gew.-% und bevorzugt etwa 2, etwa 3, etwa 4 oder etwa 5 Gew. %, bezogen auf das Gewicht der selbstklebenden Matrixschicht(en) oder der Klebeschicht.

21. Verwendung nach einem der vorhergehenden Ansprüche 13 bis 20, **gekennzeichnet durch** Aerosil* 90. Aerosil* 130. Aerosil* 150, Aerosil* 200, Aerosil* 300, Aerosil* 380, Aerosil* OX50, Aerosil* TT600, Aerosil* MOX8O, Aerosil* COK84, Aerosil* R202, Aerosil* R805, Aerosil* R812, Aerosil* 812S, Aerosil* R972 und/oder Aerosil* R974 als hochdisperses Siliziumdioxid.

22. Verwendung nach Anspruch 21, **gekennzeichnet durch** Aerosil* 200 und/oder Aerosil* R972 als hochdisperses Siliziumdioxid.

23. Verwendung nach einem der vorhergehenden Ansprüche 13 bis 22, **gekennzeichnet durch** einen Gehalt an Stabilisatoren. Emulgatoren, Verdickungsmitteln, Permeationsförderern und/oder üblichen Membransystem- oder Reservoirpflaster-Hilfsmitteln.

## Claims

1. Transdermal therapeutic system-with a cover layer impermeable to any active ingredient, with a self-adhesive matrix layer or with a plurality of matrix layers of which at least the matrix layer bared on application of the system is self-adhesive, and with a removable protective layer, the matrix layer or layers comprising one or more active ingredients and/or one or more other biologically active substances and highly disperse silica, the system comprising silica to enhance skin permeation and the active ingredient or ingredients present in the transdermal therapeutic system being an ACE inhibitor or its pharmaceutically unobjectionable salts.

2. Transdermal therapeutic system with a cover layer impermeable to any active ingredient, with one or more matrix layers comprising active ingredient and/or other biologically active substances, the matrix layer or layers being coated with a pressure-sensitive adhesive (PSA layer), and with a removable protective layer, the PSA layer comprising highly disperse silica and the active ingredient present in the transdermal therapeutic system being an ACE inhibitor or its pharmaceutically unobjectionable salts.

3. Transdermal therapeutic system according to Claim 2, **characterized in that** the system comprises silica to enhance skin permeation.

4. Transdermal therapeutic system with a cover layer impermeable to any active ingredient, with a reservoir or a reservoir layer comprising active ingredient and/or other biologically active substances, with a semipermeable membrane, with a PSA layer and with a removable protective layer, the PSA layer comprising highly disperse silica to enhance skin permeation and the active ingredient or ingredients present in the transdermal therapeutic system being an ACE inhibitor or its pharmaceutically unobjectionable salts.

5. Transdermal therapeutic system according to any one of the preceding claims, **characterized in that** the system comprises silica in an amount which in a given system (disregarding silica) corresponds to a given permeation value or is adjusted to a given permeation value.

6. Transdermal therapeutic system according to Claim 5, **characterized by** a silica content which corresponds to a maximum permeation value.

7. Transdermal therapeutic system according to any one of the preceding claims, **characterized in that** the highly disperse silica has been homogeneously incorporated in the self-adhesive matrix layer or layers or in the PSA layer.

8. Transdermal therapeutic system according to Claim 7, **characterized in that** the highly disperse silica has been incorporated by a mixture of self-adhesive matrix/silica or a mixture of pressure-sensitive adhesive/silica being allowed to swell in the presence of a liquid medium and then being formed into matrix layer or layers or PSA layer.

9. Transdermal therapeutic system according to any one of the preceding claims, **characterized by** highly disperse silica content of 0.1 to 10 wt%, in particular 2 to 5 wt% and preferably about 2, about 3, about 4 or about 5 wt%, based on the weight of the self-adhesive matrix layer or layers or of the PSA layer.

10. Transdermal therapeutic system according to any one of the preceding claims, **characterized by** Aerosil^{®} 90, Aerosil^{®} 130, Aerosil^{®} 150, Aerosil^{®} 200, Aerosil^{®} 300, Aerosil^{®} 380, Aerosil^{®} OX50, Aerosil^{®} TT600, Aerosil^{®} MOX80, Aerosil^{®} COK84, Aerosil^{®} R202, Aerosil^{®} R805, Aerosil^{®} R812, Aerosil^{®} 812S, Aerosil^{®} R972 and/or Aerosil^{®} R974 as highly disperse silica.

11. Transdermal therapeutic system according to Claim 10, **characterized by** Aerosil^{®} 200 and/or Aerosil^{®} R972 as highly disperse silica.

12. Transdermal therapeutic system according to any one of the preceding claims, **characterized by** a content of stabilizers, emulsifiers, thickening agents, permeation enhancers and/or customary membrane system or reservoir plaster adjuvants.

13. Use of highly disperse silica as a penetration enhancer in a transdermal therapeutic system with a cover layer impermeable to any active ingredient, with a self-adhesive matrix layer or with a plurality of matrix layers of which at least the matrix layer bared on application of the system is self-adhesive, and with a removable protective layer, the matrix layer or layers comprising one or more active ingredients and/or one or more other biologically active substances and highly disperse silica, the active ingredient or ingredients present in the transdermal therapeutic system being an ACE inhibitor or its pharmaceutically unobjectionable salts.

14. Use of highly disperse silica as a penetration enhancer in a transdermal therapeutic system with a cover layer impermeable to any active ingredient, with one or more matrix layers comprising active ingredient and/or other biologically active substances, the matrix layer or layers being coated with a pressure-sensitive adhesive (PSA layer), and with a removable protective layer, the PSA layer comprising highly disperse silica and the active ingredient or ingredients present in the transdermal therapeutic system being an ACE inhibitor or its pharmaceutically unobjectionable salts.

15. Use of highly disperse silica as a penetration enhancer in a transdermal therapeutic system with a cover layer impermeable to any active ingredient, with a reservoir or a reservoir layer comprising active ingredient and/or other biologically active substances, with a semipermeable membrane, with a PSA layer and with a removable protective layer, the PSA layer comprising highly disperse silica to enhance skin permeation and the active ingredient or ingredients present in the transdermal therapeutic system being an ACE inhibitor or its pharmaceutically unobjectionable salts.

16. Use according to any one of the preceding Claims 13 to 15, **characterized in that** the system comprises silica in an amount which in a given system (disregarding silica) corresponds to a given permeation value or is adjusted to a given permeation value.

17. Use according to Claim 16, **characterized by** a silica content which corresponds to a maximum permeation value.

18. Use according to any one of the preceding Claims 13 to 17, **characterized in that** the highly disperse silica has been homogeneously incorporated in the self-adhesive matrix layer or layers or in the PSA layer.

19. Use according to Claim 18, **characterized in that** the highly disperse silica has been incorporated by a mixture of self-adhesive matrix/silica or a mixture of pressure-sensitive adhesive/silica being allowed to swell in the presence of a liquid medium and then being formed into matrix layer or layers or PSA layer.

20. Use according to any one of the preceding Claims 13 to 19, **characterized by** highly disperse silica content of 0.1 to 10 wt%, in particular 2 to 5 wt% and preferably about 2, about 3, about 4 or about 5 wt%, based on the weight of the self-adhesive matrix layer or layers or of the PSA layer.

21. Use according to any one of the preceding Claims 13 to 20, **characterized by** Aerosil^{®} 90, Aerosil^{®} 130, Aerosil^{®} 150, Aerosil^{®} 200, Aerosil^{®} 300, Aerosil^{®} 380, Aerosil^{®} OX50, Aerosil^{®} TT600, Aerosil^{®} MOX8O, Aerosil^{®} COK84, Aerosil^{®} R202, Aerosil^{®} R805, Aerosil^{®} R812, Aerosil^{®} 812S, Aerosil^{®} R972 and/or Aerosil^{®} R974 as highly disperse silica.

22. Use according to Claim 21, **characterized by** Aerosil^{®} 200 and/or Aerosil^{®} R972 as highly disperse silica.

23. Use according to any one of the preceding Claims 13 to 22, **characterized by** a content of stabilizers, emulsifiers, thickening agents, permeation enhancers and/or customary membrane system or reservoir plaster adjuvants.

## Revendications

1. Système thérapeutique transdermique comprenant une couche de recouvrement imperméable à la substance active, une couche matricielle autocollante ou plusieurs couches matricielles dont au moins celle qui se trouve libre lors de l'application du système est autocollante, et une couche protectrice pouvant être retirée, la couche ou les couches matricielles contenant une ou plusieurs substances actives et/ou une ou plusieurs autres substances biologiquement actives et du dioxyde de silicium fortement dispersé, le dioxyde de silicium étant contenu dans le système pour accroître la pénétration à travers la peau, et la ou les substances actives contenues dans le système thérapeutique transdermique étant un inhibiteur de ACE ou ses sels pharmaceutiquement acceptables.

2. Système thérapeutique transdermique comprenant une couche de recouvrement imperméable à la substance active, une ou plusieurs couches matricielles ayant une teneur en substance active et/ou en d'autres substances biologiquement actives, la couche ou les couches matricielles étant revêtues d'un adhésif (couche adhésive), et une couche protectrice détachable, la couche adhésive contenant du dioxyde de silicium fortement dispersé et la substance active contenue dans le système thérapeutique transdermique étant un inhibiteur de ACE ou ses sels pharmaceutiquement acceptables.

3. Système thérapeutique transdermique suivant la revendication 2, **caractérisé en ce qu'**il contient du dioxyde de silicium pour accroître la pénétration à travers la peau.

4. Système thérapeutique transdermique comprenant une couche de recouvrement imperméable à la substance active, un réservoir ou une couche réservoir contenant une substance active et/ou d'autres substances biologiquement actives, une membrane semi-perméable, une couche adhésive et une couche protectrice détachable, la couche adhésive contenant du dioxyde de silicium hautement dispersé pour accroître la pénétration à travers la peau et la ou les substances actives contenues dans le système thérapeutique transdermique étant un inhibiteur de ACE ou ses sels pharmaceutiquement acceptables.

5. Système thérapeutique transdermique suivant l'une des revendications précédentes, **caractérisé en ce qu'**il contient du dioxyde de silicium en une quantité qui correspond, pour un système préétabli (abstraction faite du dioxyde de silicium) à une valeur de pénétration préétablie ou qui est réglée à une valeur de pénétration préétablie.

6. Système thérapeutique transdermique suivant la revendication 5, **caractérisé par** une teneur en dioxyde de silicium qui correspond à une valeur maximale de pénétration.

7. Système thérapeutique transdermique suivant l'une des revendications précédentes, **caractérisé en ce que** l'oxyde de silicium hautement dispersé a été incorporé de façon homogène à la couche ou aux couches matricielles autocollantes ou à la couche adhésive.

8. Système thérapeutique transdermique suivant la revendication 7, **caractérisé en ce que** le dioxyde de silicium hautement dispersé a été incorporé de façon telle que l'on a fait gonfler un mélange matrice autocollante/dioxyde de silicium ou un mélange adhésif/dioxyde de silicium en présence d'un milieu liquide, et on l'a ensuite moulé en couche(s) matricielle(s) ou en couche adhésive.

9. Système thérapeutique transdermique suivant l'une des revendications précédentes, **caractérisé par** une teneur en dioxyde de silicium hautement dispersé de 0,1 à 10 % en poids, en particulier de 2 à 5 % en poids et préférentiellement d'environ 2, d'environ 3, d'environ 4 ou d'environ 5 % en poids, par rapport au poids de la couche ou des couches matricielles autocollantes ou de la couche adhésive.

10. Système thérapeutique transdermique suivant l'une des revendications précédentes, **caractérisé par** la présence de Aerosil*90, Aerosil*130, Aerosil*150, Aerosil*200, Aerosil*300, Aerosil*380, Aerosil*OX50, Aerosil*TT600, Aerosil*MOX80, Aerosil*COK84, Aerosil*R202, Aerosil*R805, Aerosil*R812, Aerosil*812S, Aerosil*R972 et/ou Aerosil*R974 comme dioxyde de silicium hautement dispersé.

11. Système thérapeutique transdermique suivant la revendication 10, **caractérisé par** la présence de Aerosil*200 et/ou Aerosil*R972 comme dioxyde de silicium hautement dispersé.

12. Système thérapeutique transdermique suivant l'une des revendications précédentes, **caractérisé par** une teneur en agents stabilisants, émulsifiants, agents épaississants, substances facilitant la pénétration et/ou substances auxiliaires usuelles pour système à membrane ou emplâtre-réservoir.

13. Utilisation de dioxyde de silicium hautement dispersé comme substance facilitant la pénétration dans un système thérapeutique transdermique comprenant une couche de recouvrement imperméable à la substance active, une couche matricielle autocollante ou plusieurs couches matricielles dont au moins celle qui se trouve libre lors de l'application du système est autocollante, et une couche protectrice détachable, la couche ou les couches matricielles contenant une ou plusieurs substances actives et/ou une ou plusieurs autres substances biologiquement actives et du dioxyde de silicium hautement dispersé, la substance active ou les substances actives contenues dans le système thérapeutique transdermique consistant en un inhibiteur de ACE ou ses sels pharmaceutiquement acceptables.

14. Utilisation de dioxyde de silicium hautement dispersé comme substance facilitant la pénétration dans un système thérapeutique transdermique comprenant une couche de recouvrement imperméable à la substance active, une ou plusieurs couches matricielles ayant une teneur en substance active et/ou en d'autres substances biologiquement actives, la ou les couches matricielles étant revêtues d'un adhésif (couche adhésive), et une couche protectrice détachable, la couche adhésive contenant du dioxyde de silicium hautement dispersé et la ou les substances actives contenues dans le système thérapeutique transdermique étant un inhibiteur de ACE ou ses sels pharmaceutiquement acceptables.

15. Utilisation de dioxyde de silicium hautement dispersé comme substance facilitant la pénétration dans un système thérapeutique transdermique comprenant une couche de recouvrement imperméable à la substance active, un réservoir ou une couche réservoir ayant une teneur en substance active et/ou en d'autres substances biologiquement actives, une membrane semi-perméable, une couche adhésive et une couche protectrice détachable, la couche adhésive contenant du dioxyde de silicium hautement dispersé pour accroître la pénétration à travers la peau, et la ou les substances actives contenues dans le système thérapeutique transdermique étant un inhibiteur de ACE ou ses sels pharmaceutiquement acceptables.

16. Utilisation suivant l'une des revendications 13 à 15 précédentes, **caractérisée en ce que** le système contient du dioxyde de silicium en une quantité qui correspond, pour un système préétabli (abstraction faite du dioxyde de silicium) à une valeur de pénétration préétablie ou qui est réglée à une valeur de pénétration préétablie.

17. Utilisation suivant la revendication 16, **caractérisée par** une teneur en dioxyde de silicium qui correspond à une valeur maximale de pénétration.

18. Utilisation suivant l'une des revendications 13 à 17 précédentes, **caractérisée en ce que** le dioxyde de silicium hautement dispersé a été incorporé de façon homogène à la couche ou aux couches matricielles autocollantes ou à la couche adhésive.

19. Utilisation suivant la revendication 18, **caractérisée en ce que** le dioxyde de silicium hautement dispersé a été incorporé de façon telle que l'on a fait gonfler un mélange matrice autocollante/dioxyde de silicium ou un mélange adhésif/dioxyde de silicium en présence d'un milieu liquide et on l'a ensuite moulé en une ou plusieurs couches matricielles ou une couche adhésive.

20. Utilisation suivant l'une des revendications 13 à 19 précédentes, **caractérisée par** une teneur en dioxyde de silicium hautement dispersé de 0,1 à 10 % en poids, en particulier de 2 à 5 % en poids et préférentiellement d'environ 2, d'environ 3, d'environ 4 ou d'environ 5 % en poids, par rapport au poids de la couche ou des couches matricielles autocollantes ou de la couche adhésive.

21. Utilisation suivant l'une des revendications 13 à 20 précédentes, **caractérisée par** la présence de Aerosil*90, Aerosil*130, Aerosil*150, Aerosil*200, Aerosil*300, Aerosil*380, Aerosil*OX50, Aerosil*TT600, Aerosil*MOX80, Aerosil*COK84, Aerosil*R202, Aerosil*R805, Aerosil*R812, Aerosil*812S, Aerosil*R972 et/ou Aerosil*R974 comme dioxyde de silicium hautement dispersé.

22. Utilisation suivant la revendication 21, **caractérisée par** la présence de Aerosil*200 et/ou Aerosil*R972 comme dioxyde de silicium hautement dispersé.

23. Utilisation suivant l'une des revendications 13 à 22 précédentes, **caractérisée par** une teneur en agents stabilisants, émulsifiants, agents épaississants, substances facilitant la pénétration et/ou substances auxiliaires usuelles pour système à membrane ou emplâtre-réservoir.
